# FASCICULE DE BREVET EUROPEEN

(11) **EP 2 785 187 B1**
(45) Date de publication et mention de la délivrance du brevet: **05.10.2016**
(21) Numéro de dépôt: 12816696.4
(22) Date de dépôt: 03.12.2012
(51) Int. Cl.: A01N 63/04, C12N 1/14, C05F 17/00, C05F 11/08, C12N 1/22

(54) **PROCÉDÉ DE MULTIPLICATION DE MICRO-ORGANISMES PHYTO-BENEFIQUES**
VERFAHREN ZUM VERVIELFÄLTIGEN PFLANZENFÖRDERNDER MIKROORGANISMEN
METHOD FOR MULTIPLYING PHYTOBENEFICAL MICROORGANISMS

(30) Priorité: 02.12.2011 FR 1161099
(43) Date de publication de la demande: 08.10.2014
(73) Titulaire: Florentaise, 44850 St Mars Du Desert (FR); Nixe, 06560 Valbonne (FR)
(72) Inventeur: COWPER, Jérôme, R., F-47600 Moncrabeau (FR); CANAGUIER, Renaud, H., 06370 Mouans Sartoux (FR); REYNAUD, Hélène, L., F-83440 Tanneron (FR)
(74) Mandataire: Nevant, Marc
(86) Numéro de dépôt international: PCT/FR2012/052776
(87) Numéro de publication internationale: WO 2013/079887

(56) Documents cités:
- EP-A1- 0 324 689
- EP-A1- 0 947 130
- EP-A1- 1 400 586
- EP-A1- 1 876 232
- EP-A2- 1 281 753
- WO-A1-2009/083819
- JP-A- 2006 035 150
- TENGERDY ET AL: "Solid substrate fermentation", TRENDS IN BIOTECHNOLOGY, ELSEVIER PUBLICATIONS, CAMBRIDGE, GB, vol. 3, no. 4, 1 avril 1985 (1985-04-01), pages 96-99, XP023595312, ISSN: 0167-7799, DOI: 10.1016/0167-7799(85)90092-7 [extrait le 1985-04-01]
- Akinori Noguchi and Seiichiro Isobe: "New Food proteins, Extrusion processes and Products in Japan" In: "Proceedings of the World Congress on Vegetable Protein Utilization in Human Food and Animal Foodstuffs", 1989, American Oil Chemist's Society, Champaign, Illinois, XP009167695, ISBN: 0-935315-25-X pages 375-381, page 380 - page 381
- R. RAMA RAO ET AL: "Biomass protein formation by filamentous fungi on woody substrates", BIOTECHNOLOGY LETTERS, vol. 6, no. 7, 1 juillet 1984 (1984-07-01) , pages 461-464, XP055029301, ISSN: 0141-5492, DOI: 10.1007/BF00129310
- Sevastianos Roussos: "CROISSANCE DE TRICHODERMA HARZIANUM PAR FERMENTATION EN MILIEU SOLIDE: PHYSIOLOGIE, SPORULATION ET PRODUCTION DE CELLULASES", 1 janvier 1987 (1987-01-01), XP055029269, ISBN: 978-2-70-990857-3 page 125 - page 131

## Description

### Domaine de l'invention

La présente invention concerne le domaine des supports de culture. Plus particulièrement, l'invention concerne un procédé de multiplication de microorganismes phyto-bénéfiques, notamment de microorganismes fongiques.

### Arrière-plan technologique

L'enrichissement des terreaux industriels en flore microbienne utile présente un intérêt, soit pour assainir les substrats de culture des plantes, soit pour en améliorer la fertilité.

Différents microorganismes et différentes bactéries sont utilisés comme agents de biocontrôle des plantes.

Les microorganismes qui favorisent la croissance des plantes sont dénommés PGPM (de l'anglais « plant growth promoting micro-organisms »). Les champignons mycorhiziens symbiotiques et certaines espèces de *Trichoderma* mutualistes avec les plantes, font partie de cette catégorie. Les bactéries promotrices de croissance et spécifiques de la rhizosphère sont dénommées PGPR (de l'anglais « plant growth promoting rhizobacteria »). Cette catégorie comprend, par exemple, les *Pseudomonas* et les *Bacillus* utiles.

Le genre *Trichoderma* est un groupe agronomiquement important car il comprend des champignons agents de biocontrôle dont le mode d'action a fait l'objet de nombreux travaux. Des études récentes font en effet état d'une capacité de ce champignon filamenteux à intervenir selon divers mécanismes : mycoparasitisme, antagonisme (compétition), antibiose (production d'antibiotiques), stimulation racinaire, stimulation de la croissance par solubilisation de minéraux fertilisants, stimulation des défenses naturelles des plantes.

Les champignons du genre *Trichoderma* appartiennent au phylum *Ascomycota,* classe des Ascomycetes, famille des Hypocreales. Ce sont des champignons microscopiques dont il existe des espèces terrestres et des espèces marines. On en trouve dans les bois en décomposition, les résidus végétaux et dans tous les sols (humus forestiers, terres agricoles) (de 10 à 10000 propagules/g dans les sols tempérés ou tropicaux). Ils colonisent les racines des plantes herbacées et ligneuses sans aucun dommage. En outre, ce champignon peut pénétrer dans les racines et favoriser le développement, la nutrition et la résistance aux maladies. L'espèce atroviride est l'une des espèces courantes de *Trichoderma.*

La colonisation des racines par *Trichoderma sp.* peut augmenter la croissance et le développement des racines, le rendement des cultures, la résistance aux stress abiotiques et l'absorption et l'utilisation des nutriments (Harman G.E. et al., Nature Reviews / Microbiology, 2, 43-56, 2004).

Lorsqu'ils existent, les effets de stimulation de croissance proviennent de l'action directe des *Trichoderma* sur les plantes et ne sont pas directement liés aux antagonismes avec les pathogènes. Ces effets sont visibles aussi bien sur des substrats de culture non désinfectés que sur des substrats stériles. Les mécanismes de stimulation de croissance sont mal élucidés et pourraient être dus à la suppression des dommages oxydatifs sur les racines, à la sécrétion de facteurs de croissance par le champignon, à l'inhibition de la microflore gênante et à l'amélioration du transport des micronutriments.

Les effets sont inégaux d'une souche à l'autre. Certaines souches possèdent des effets stimulateurs de croissance mais d'autres ont des effets inhibiteurs (par exemple, Trichoderma viride RF1, J.G. Menzies, Plant Pathology, 42, 784-791, 1993).

Les propriétés antagonistes des *Trichoderma* sont mieux documentées que les propriétés stimulatrices. Le potentiel antagoniste des *Trichoderma* vis-à-vis de nombreux champignons du sol pathogènes des plantes a été découvert dans les années 1930 (Weindling R., Trichoderma lignorum as a parasite of other soil fungi, Phytopathology, 22, 837-845, 1932). L'application la plus évidente est la lutte biologique en agriculture (biocontrôle), y compris en agriculture biologique où le règlement (CE) n° 2092/91 prévoit cette utilisation.

D'une manière générale, il existe deux manières d'enrichir un milieu en microorganismes :
- Par dilution dans le milieu d'une quantité suffisante de microorganismes à partir d'une préparation concentrée ; ou
- Par inoculation du milieu et multiplication *in situ* des microorganismes.

La première technique fait appel à une production de microorganismes dans un système séparé. Elle s'applique bien aux microorganismes qui possèdent des formes de résistance stables (par exemple, spores, conidies ou chlamydospores). La technique consiste à diluer le micro-organisme obtenu séparément dans un milieu qui doit être compatible, et qui peut être susceptible d'aider à sa régénération lorsque qu'une condition favorable apparaît (par exemple, hydratation, réchauffement du milieu). Une telle technique est décrite par exemple dans la demande de brevet US 2004/0136964A1. Ce document concerne un substrat contenant une souche de *Trichoderma asperellum* pour le contrôle biologique du *Fusarium* et du *Rhizoctonia,* ledit substrat étant obtenu à partir d'un mélange constitué de déchets, de boues d'épuration, de tourbe, d'écorce ou de compost.

La seconde technique s'applique aux microorganismes capables de se multiplier dans le milieu jusqu'à l'épuisement des ressources nutritives ou la dégradation des conditions vitales, pour donner ensuite des formes dormantes, dites de résistance. Les formes de résistance qui peuvent régénérer les microorganismes entrent dans la catégorie des propagules. Cette seconde technique permet d'obtenir un milieu riche en propagules stables. L'épuisement des matières facilement fermentescibles rend le milieu peu propice au développement d'autres microorganismes. L'enrichissement par la multiplication *in situ* peut s'appliquer à des bactéries, des levures et des champignons, susceptibles de vivre dans les milieux à base de fibre de bois. La multiplication concerne aussi la catégorie des champignons mycorhiziens qui sont aussi saprophytes. La multiplication peut aussi concerner les champignons mycorhiziens symbiotiques des plantes à condition de les cultiver sur des racines hôtes.

Contrairement à la technique par dilution, la multiplication *in situ* ne demande pas de grandes quantités de microorganismes produits en conditions stériles. La multiplication est obtenue grâce à la consommation de substances nutritives contenues ou ajoutées dans le substrat. La multiplication est par principe, aérobie. Le milieu est considéré comme « activé », lorsque des substances nutritives spécifiques sont ajoutées pour favoriser la multiplication. Les substances nutritives spécifiques ajoutées au milieu « activé » sont partiellement ou totalement consommées à l'issue de la phase de multiplication. Cela se traduit par une perte de masse et la production de métabolites volatils, en particulier de dioxyde de carbone (CO₂) et d'eau (H₂O).

La technique de multiplication *in situ* comprend une phase de préparation du milieu, avec si nécessaire une désinfection, l'ajout d'une quantité suffisante d'inoculum primaire de microorganismes, la période de multiplication des microorganismes, l'apparition des formes de résistances et la maturation. Chaque étape possède une durée variable selon les conditions et les microorganismes employés. La multiplication peut se faire par cycles successifs d'enrichissement, le produit d'un cycle de multiplication servant à inoculer une quantité plus importante de milieu. Cycle après cycle, les milieux de multiplication successifs peuvent devenir de plus en plus simples et les conditions de multiplication de moins en moins sévères. Cela permet d'obtenir un bon rendement sans utiliser de techniques lourdes de production. La technique par étape améliore le rendement en biomasse et réduit la perte de substrat consommé pour le développement des microorganismes.

La multiplication *in situ* permet également de réduire les microorganismes gênants pendant la production du terreau grâce à l'instauration d'une compétition. Les propagules obtenues par la technique de multiplication sont plus stables que des microorganismes additionnés postérieurement à la fabrication du terreau. Le terreau obtenu par le processus de multiplication est susceptible de renfermer des métabolites secondaires libérés lors de la fermentation. Les métabolites secondaires de certaines espèces de champignons (*Trichoderma*) et bactéries (*Pseudomonas*) sont utiles aux plantes, soit parce qu'ils assainissent et détoxiquent les substrats, soit parce qu'ils stimulent la croissance.

Dans la pratique, les microorganismes utiles aux plantes vivent dans la rhizosphère ou à proximité immédiate de la rhizosphère et bénéficient pour leur nutrition des exsudats racinaires et des résidus végétaux. Certains microorganismes, tels les *Trichoderma,* vivent de manière saprophyte dans le sol ou en surface, sur des restes de végétaux morts.

Différentes sortes de fibres sont favorables à la croissance des microorganismes ; on peut citer notamment la fibre de bois, la fibre de palme et les différentes fibres végétales utilisées dans les applications agricoles, textiles ou d'isolation. Des matériaux aérés et fibreux, d'origine végétale, en copeaux, feuilles ou pailles conviennent aussi.

Les demandes de brevet EP-A-0 147 349 et FR-A-2 776 470 décrivent un milieu de culture à base de fibres de bois obtenues par cuisson à la vapeur et défibrage, selon des procédés rencontrés aussi dans l'industrie papetière et l'industrie des panneaux de bois agglomérés. Comme indiqué dans la demande de brevet FR-A-2 776 470, le défibrage est réalisé de manière conventionnelle à l'aide d'un défibreur à disques, à partir de copeaux de bois dont on « extrait » les fibres par cisaillement à température élevée.

Il a toutefois été constaté par la demanderesse, à la suite de nombreux essais, que les procédés décrits dans les documents précités avaient tendance à provoquer une cuisson du bois s'apparentant à un début de torréfaction ou à une thermo-stabilisation, ayant pour conséquence une réduction de l'hydrophilie et de la capacité de rétention d'eau des fibres de bois produites. Ces inconvénients rendent les fibres de bois peu propices à une multiplication adéquate des microorganismes.

Il est donc souhaitable de pouvoir disposer de fibres de bois susceptibles de favoriser la multiplication de microorganismes, notamment du genre *Trichoderma,* qui sont exemptes des inconvénients mentionnés ci-dessus.

Il est également souhaitable de pouvoir disposer d'un milieu de culture qui soit adapté à la multiplication de tels microorganismes, et qui permette l'obtention d'un support de culture propice à la culture des plantes.

### Description de l'invention

Il a maintenant été trouvé, et c'est le fondement de l'invention, qu'il est possible de multiplier des microorganismes du genre *Trichoderma* à l'aide d'un milieu de culture particulier contenant des fibres de bois pour préparer un support de culture des plantes.

Ainsi, l'invention concerne selon un premier aspect un procédé de multiplication *in situ* de microorganismes du genre *Trichoderma* qui comprend des étapes de désinfection, de multiplication et d'amplification par étapes :
- la désinfection de la fibre de bois au cours de sa fabrication donne immédiatement un substrat propice à la culture des *Trichoderma,* en une seule étape. On peut faire pousser le *Trichoderma* sur des copeaux de bois, mais une stérilisation est nécessaire après fabrication des copeaux. Dans le cas de la fibre, fabrication et stérilisation sont simultanées ;
- la multiplication *in situ* du champignon *Trichoderma* permet d'ensemencer le support de culture des plantes en micro-organismes utiles et de limiter le taux de contaminants. La multiplication *in situ* permet de n'apporter qu'une quantité faible de micro-organismes par rapport à celle qui sera contenue dans le produit fini. Les micro-organismes se multiplient spontanément grâce à la composition particulière du support de culture ;
- l'amplification du champignon *Trichoderma* par étapes successives évite la désinfection de masses importantes de matière. Les conditions de multiplication sont de moins en moins sévères au fil des étapes. Cette méthode diffère de l'inoculation en masse de la totalité du support de culture des plantes.

Selon un mode de réalisation de l'invention, le procédé comprend :
- l'ensemencement, par une suspension mère de propagules de *Trichoderma,* d'un milieu de culture stérilisé contenant entre environ 25% et environ 50% en masse de fibres de bois ainsi que des substances nutritives, de l'eau et un correcteur de pH, lesdites fibres ayant un taux d'air en volume compris dans la gamme d'environ 55% à environ 90%, ledit milieu étant exempt de contaminants fongiques et possédant un taux de contaminants bactériens inférieur ou égal à 10² UFC/g ;
- la culture du milieu de culture ensemencé pour obtenir un inoculum primaire dans lequel le facteur de multiplication des propaqules atteint 2.10⁴ à 10⁵.

Il est particulièrement important de supprimer les contaminants fongiques du milieu de culture et d'en réduire le taux de bactéries en dessous de 10² UFC/g. L'élimination des *Aspergillus* et des *Trichoderma* autochtones est en particulier nécessaire car certaines souches de ces champignons peuvent entrer en compétition avec les microorganismes inoculés. Le milieu désinfecté est particulièrement propice à l'inoculation et au développement des microorganismes utiles. Les microorganismes inoculés ne rencontrent pas d'organismes compétiteurs et peuvent se développer librement et rapidement. De préférence, le milieu de culture est stérilisé par autoclavage dans les conditions habituelles (121°C, environ 30 min) avant d'être ensemencé puis incubé.

De manière avantageuse, la concentration finale en propagules de l'inoculum primaire se situe dans la gamme d'environ 10⁸ à environ 10¹⁰ propagules/g.

De manière également avantageuse, la culture de *Trichoderma* comprend une période d'incubation d'au moins deux semaines.

Selon un autre mode de réalisation, le procédé conforme à l'invention comprend en outre :
- la préparation d'un second milieu de culture contenant des fibres de bois désinfectées, notamment par chauffage, lesdites fibres de bois ayant un taux d'air en volume compris dans la gamme d'environ 55% à environ 90% ;
- l'inoculation dudit milieu de culture avec 0,001 % à 5 % en masse de l'inoculum primaire ;
- la culture du milieu de culture ainsi inoculé pour obtenir un inoculum secondaire dans lequel la multiplication des propagules atteint 10³ à 10⁵. De manière avantageuse, la concentration finale en propagules dans l'inoculum secondaire se situe dans la gamme d'environ 10⁷ à environ 10⁹ propagules/g.

L'invention concerne également, dans un second aspect, un procédé de préparation d'un inoculum secondaire de *Trichoderma* qui comprend :
- la préparation d'un milieu de culture contenant des fibres de bois désinfectées notamment par chauffage, lesdites fibres de bois ayant un taux d'air en volume compris dans la gamme d'environ 55% à environ 90% ;
- l'inoculation dudit milieu de culture avec 0,001 % à 5 % d'un inoculum primaire de *Trichoderma,* susceptible d'être obtenu comme décrit ci-dessus, dont la concentration en propagules se situe dans la gamme allant de 10⁸ à 10¹⁰ propagules/g ;
- la culture du milieu de culture ainsi inoculé pour obtenir un inoculum secondaire dont la concentration finale en propagules se situe dans la gamme allant d'environ 10⁷ à environ 10⁹ propagules/g.

De manière avantageuse, la culture du milieu inoculé avec l'inoculum primaire comprend une période d'incubation d'au moins deux semaines.

De préférence, le milieu de culture utilisé pour la préparation de l'inoculum primaire, et celui utilisé pour la préparation de l'inoculum secondaire, comprennent chacun des fibres de bois ayant un taux d'air en volume compris dans la gamme allant d'environ 60% à environ 85%, de préférence d'environ 70% à environ 85%.

De manière avantageuse, les fibres de bois contenues dans les différents milieux de culture mentionnés ci-dessus sont obtenues par extrusion de copeaux de bois, en particulier de plaquettes de bois, dans une extrudeuse, de préférence une extrudeuse bivis. Dans un mode de réalisation de l'invention les copeaux de bois sont extrudés en présence de son de blé.

De manière tout à fait avantageuse, la souche de *Trichoderma* que l'on cherche à multiplier est la souche *Trichoderma atroviride* MUCL45632.

Un milieu de culture tel que défini ci-dessus en référence au premier aspect ou au second aspect de l'invention est également décrit ici.

### Conditions de production de Trichoderma

Le milieu de culture est constitué de matières fermentescibles, plus particulièrement, des glucides, des lipides et des protéines. Des minéraux sont nécessaires, tels le phosphore, le potassium, le magnésium et le soufre. Il existe aussi certains facteurs qui favorisent la croissance et la sporulation. Le calcium, le cobalt, le fer, le manganèse et le zinc (Mandels et Reese, 1957) sont cités comme des éléments favorables à la croissance et à la production de cellulase par *Trichoderma viride.*

Il apparaît que les champignons *Trichoderma* se développent dans des milieux acides. L'acidification rend le milieu de culture sélectif pour *Trichoderma* en inhibant la croissance des bactéries. Dans ces conditions, l'ajout d'un correcteur de pH dans le milieu est avantageux ; la correction peut être de l'ordre de 0,01 à environ 6 unités de pH.

Le brevet GB1573850 décrit la production de *Trichoderma* par culture sur des céréales acidifiées à pH < 4,5 et enrichies en cuivre, dans des sacs en plastique étanches qui sont périodiquement remplis d'air et vidés de leur atmosphère. Il est décrit aussi le fractionnement périodique des lits de culture afin de favoriser l'homogénéité du milieu.

La demande de brevet EP-A-1 876 232, qui concerne notamment la souche de *Trichoderma atroviride* MUCL45632, décrit un milieu de culture approprié à la production de propagules de ce champignon : « Le milieu de culture est essentiellement constitué d'un substrat d'origine végétale riche en polyholosides tels l'amidon, l'hémicellulose et la cellulose. Ce substrat pourra être constitué de manière non limitative, de graines de céréales (orge, avoine) ou de légumineuses (soja, lupin, fèves), de sous-produits de l'industrie des céréales (son de céréales, germes de blé, coques de riz), de tourteaux d'extraction d'oléagineux (tournesol, coton), de déchets de l'industrie du sucre et de l'industrie de l'amidon (pulpe de betterave, bagasse de canne à sucre), de paille ou de copeaux de bois. Le substrat végétal doit être broyé grossièrement pour former une masse perméable à l'eau et à l'air. Le milieu pourra être complémenté en éléments minéraux utiles à la croissance du champignon : nitrate, ammonium, ortho-phosphate, potassium, magnésium, calcium et oligo-éléments. Le pH sera avantageusement acidifié à l'aide d'un acide de préférence minéral, comme l'acide chlorhydrique, l'acide sulfurique ou l'acide ortho-phosphorique, entre pH 2,7 et pH 4 et de préférence entre 2,7 et 3,3 ». Ces conditions conviennent d'une manière générale à d'autres *Trichoderma,* mais l'homme du métier adaptera si nécessaire les conditions de culture pour obtenir un rendement optimal en propagules.

La culture de *Trichoderma* peut se faire dans différents contenants : sacs, boîtes, caisses ou cuves, en matériaux adaptés, tels l'acier inoxydable, le verre et les matières plastiques. Le bois convient aussi, mais il est susceptible d'être attaqué par les *Trichoderma* et altéré par l'humidité.

La température de culture des *Trichoderma* dépend des souches. La plupart des *Trichoderma* poussent à partir de 12°C et jusqu'à 35°C. Les températures optimales de croissance se situent dans la gamme de 20 à 28°C. La consommation des glucides par les champignons provoque un échauffement du milieu (17 kJ/g de glucide oxydé). Cet échauffement doit être limité, car au-delà de 36°C, les champignons *Trichoderma* commencent à mourir. L'échauffement est contrôlable de différentes manières :
- En limitant la quantité de glucides facilement oxydables. Cela peut se faire en contrôlant la quantité de sucres simples introduits au chargement, ou en utilisant des glucides complexes, dénommés aussi fibres, nécessitant une succession d'hydrolyses enzymatiques avant d'être disponibles pour l'oxydation. L'amidon (α-glucane) qui s'hydrolyse rapidement est pratiquement considéré comme un sucre simple. La cellulose du bois (β-glucane) est un sucre complexe qui s'oxyde lentement.
- En contrôlant l'atmosphère autour du milieu de culture. La réduction de l'oxygène et l'augmentation du taux de dioxyde de carbone limitent l'activité métabolique des champignons. Cela peut être obtenu en plaçant le milieu de culture dans une enceinte fermée et en freinant les échanges gazeux de cette enceinte avec l'air extérieur.
- En contrôlant la quantité d'eau initiale et l'évaporation d'eau en cours de culture. L'évaporation d'eau est une des méthodes d'abaissement de température d'un milieu de culture. L'évaporation est d'autant plus intense que la pression partielle d'eau dans l'atmosphère (hygrométrie) est faible. La réduction de pression partielle s'obtient, en particulier, en augmentant les échanges gazeux avec l'air extérieur, en opposition avec le principe de limitation des échanges gazeux exposé ci-dessus.
- En favorisant les échanges de température par la maximisation de la surface des lits de culture au détriment du volume, c'est à dire, par exemple, en utilisant des contenants de forme aplatie, tels des sacs plats ou des matelas.
- En refroidissant l'atmosphère ou le milieu de culture.

Les champignons consomment de l'oxygène au cours de leur croissance (747 L O₂ / kg de glucides oxydés). L'aération est essentielle, mais elle peut être limitée au strict nécessaire pour obtenir une croissance régulière. L'air est un vecteur de contaminants, il est donc nécessaire de ventiler avec un air purifié ou de placer des systèmes qui limitent la propagation des contaminants par la voie aérienne.

### Fibres de bois

Les fibres de bois utilisées dans le cadre de la présente invention sont volumineuses et aérées, ce qui permet la bonne respiration des microorganismes au sein de la matière ; les fibres possèdent un taux d'air en volume compris dans la gamme allant d'environ 55% à environ 90%, de préférence dans la gamme allant d'environ 60% à environ 85%, de préférence encore dans la gamme allant d'environ 70% à environ 85%. Ces fibres de bois sont avantageusement obtenues par extrusion de copeaux de bois, notamment de plaquettes de bois telles que des plaquettes de résineux de qualité papetière issues de l'aubier du bois. L'extrusion est réalisée à l'aide d'une extrudeuse bivis. Les fibres de bois obtenues possèdent les caractéristiques physico-chimiques suivantes :
- une densité apparente sèche comprise dans la gamme allant d'environ 30 à environ 100 kg/m³, de préférence dans la gamme allant d'environ 40 à environ 100 kg/m³, de préférence encore dans la gamme allant d'environ 50 à environ 100 kg/m³ ;
- une porosité comprise dans la gamme allant d'environ 88 à environ 98% en volume ;
- une rétention en air comprise dans la gamme allant d'environ 55 à environ 80%, de préférence dans la gamme allant d'environ 55 à environ 70% en volume ;
- une rétention en eau comprise dans la gamme allant d'environ 18 à environ 33%, de préférence dans la gamme allant d'environ 23 à environ 33% en volume ;
- une conductivité électrique inférieure ou égale à environ 0,5 mS/cm, de préférence dans la gamme allant d'environ 0,2 à environ 0,5% mS/cm, de préférence encore dans la gamme allant d'environ 0,3 à environ 0,5 mS/cm.

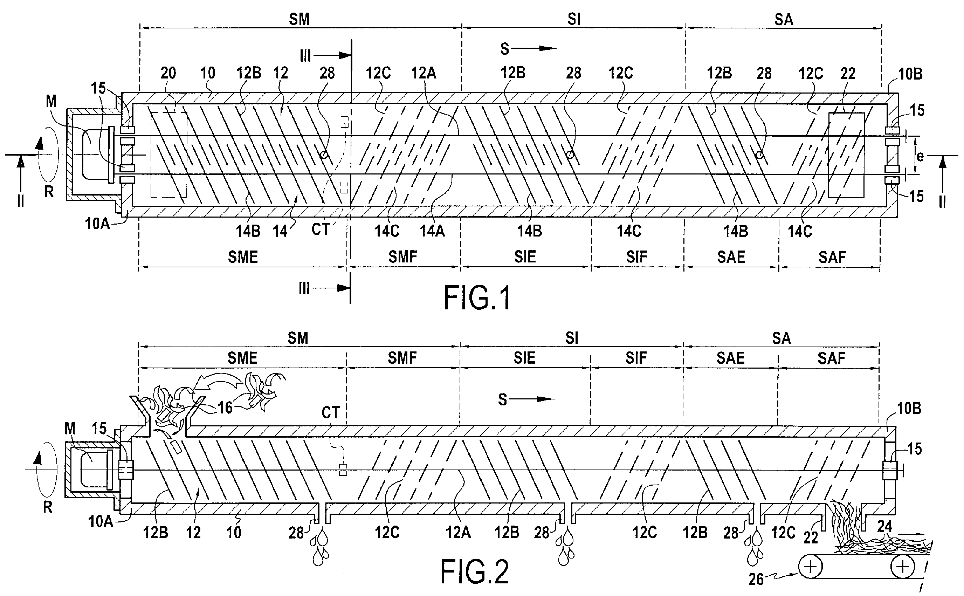

Les fibres de bois utilisées dans le cadre de la présente invention sont susceptibles d'être obtenues par le procédé décrit dans la demande de brevet FR 11 58555 déposée le 26 septembre 2011, et qui est décrit ci-après en référence aux figures 1 à 3 annexées :
- la figure 1 est une vue schématique de dessus d'une installation de défibrage permettant la mise en oeuvre dudit procédé, dans laquelle la paroi du fourreau est coupée ;
- la figure 2 est une vue schématique correspondant à une vue de l'installation de la figure 1 en coupe dans le plan II-II de la figure 1, les parties de la vis 14 présente dans ce plan étant omises ;
- la figure 3 est une coupe selon la ligne III-III de la figure 1.

Selon ce procédé, on introduit des copeaux de bois (16) dans un fourreau de défibrage (10) contenant deux vis parallèles (12, 14) entraînées en rotation de manière à engrener l'une dans l'autre par leurs filets respectifs (12B, 14B), lesdits filets présentant successivement, dans le sens (S) allant de l'amont vers l'aval, au moins une série amont et une série aval de tronçons (SM, SI, SA) comprenant chacune une zone amont d'entraînement (SME, SIE, SAE) dans laquelle les copeaux sont entraînés vers l'aval et une zone aval de freinage (SMF, SIF, SAF) à travers laquelle les copeaux sont forcés sous l'effet d'entraînement procuré par la zone amont, les copeaux étant ainsi entraînés dans le fourreau (10) en étant freinés dans les zones de freinage et en étant transformés en fibres (24) que l'on récupère en sortie du fourreau de défibrage (10), la durée de séjour des copeaux dans le fourreau (10) étant comprise entre 15 et 80 secondes, une pression au moins sensiblement égale à 90 bars étant assurée à l'amont de la zone aval de freinage (SMF) de la série amont (SM), de telle sorte qu'une température comprise entre 120°C et 150°C soit atteinte dans le fourreau (10) sans apport extérieur de chaleur.

Dans un mode de réalisation de ce procédé, la durée de séjour des copeaux dans le fourreau (10) est comprise entre 25 et 60 secondes.

Dans un autre mode de réalisation de ce procédé, on fait tourner les vis (12, 14) à une vitesse comprise entre 250 et 400 tours par minute, de préférence entre 300 et 380 tours par minute. Cette vitesse de rotation s'avère être assez élevée pour assurer un défibrage efficace des copeaux. La longueur des vis et la charge du fourreau en copeaux de bois peuvent alors être déterminées pour assurer le temps de séjour souhaité des fibres dans le fourreau. Par exemple, on choisira des vis dont la longueur est comprise entre 1600 mm et 3000 mm. Si la longueur des vis excède 2000 ou 2300 mm, il est avantageux de prévoir qu'elles comportent, en plus des séries amont et aval de tronçons, une série intermédiaire, ayant également une zone amont d'entraînement et une zone aval de freinage.

Dans un autre mode de réalisation de ce procédé, dans les zones amont d'entraînement (SME, SIE, SAE), on met les copeaux au contact de filets à pas direct, tandis que, dans les zones aval de freinage (SMF, SIF, SAF), on met les copeaux au contact de filets à pas inverse.

Dans un autre mode de réalisation de ce procédé, dans les zones aval de freinage (SMF, SIF, SAF), on fait passer les copeaux à travers des encoches (12C, 14C) que présentent les filets (12B, 14B).

Dans un autre mode de réalisation de ce procédé, chaque filet à pas inverse de la zone aval de freinage (SAF) de la série aval (SA) comporte de 2 à 6 encoches et le rapport R_{sd} entre le débit de fibres sortantes et la somme des sections des encoches d'un tel filet est compris entre 60 et 80 mm²/m³, de préférence entre 70 et 75 mm²/m³h⁻¹.

Ainsi, les encoches étant déterminées comme indiqué ci-dessus, l'alimentation du fourreau en copeaux qui détermine la charge du fourreau en copeaux est réalisée de telle sorte que le rapport entre la somme des sections des encoches d'un filet et le débit de fibres sortantes soit dans la plage indiquée ci-dessus. Dans ces conditions, une pression d'au moins 90 bars est obtenue de manière simple, sans nécessiter une structure de fourreau complexe. Le débit de fibres sortantes est déterminé en mesurant, selon la norme NF EN 12580, le volume de fibres recueillies à la sortie du fourreau exprimé en m³, en une heure.

L'installation représentée sur les figures comprend un fourreau 10 dans lequel sont disposées deux vis 12, 14 engrenant l'une dans l'autre. En effet, l'entraxe e entre les deux vis est inférieur au diamètre extérieur de leurs filets. Les arbres 12A et 14A des vis 12 et 14 sont entraînés en rotation par un moteur M et supportés en rotation par des paliers, tels que les paliers 15.

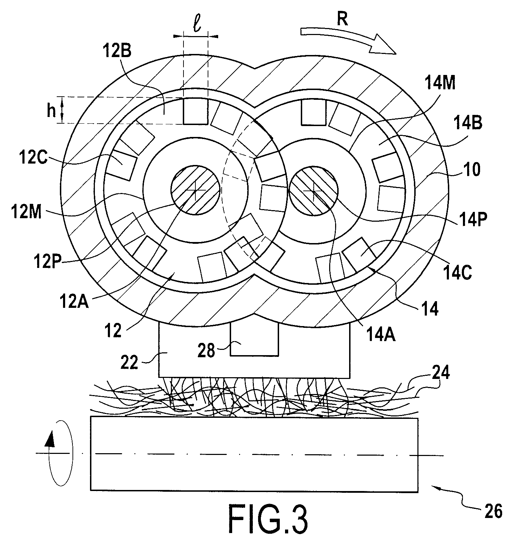

Comme on le voit mieux sur la figure 3, la paroi externe du fourreau a la forme de deux portions de cylindres sécantes adaptées, chacune, au diamètre des vis 12 et 14. Le fourreau présente, de préférence sur toute sa longueur, un capot ouvrant formant l'une de ses parois longitudinales pour permettre sa maintenance et son débourrage, si nécessaire.

Les copeaux ou plaquettes 16 devant être défibrés, c'est-à-dire réduits en fibres, sont chargés dans le fourreau par une alimentation 20, située à l'extrémité amont 10A du fourreau et ayant par exemple la forme d'une trémie située sur la face supérieure du fourreau, dans laquelle les copeaux sont acheminés par tout moyen approprié, par exemple par un convoyeur à vis, non représenté.

A son extrémité aval 10B, le fourreau présente une sortie 22. Il s'agit par exemple d'une goulotte située à la face inférieure du fourreau et laissant tomber les fibres 24 par gravité sur un convoyeur à bande 26. Ce convoyeur peut être équipé d'un tunnel (non représenté), ventilé par un gaz tel que de l'air, de préférence filtré, pour progressivement refroidir les fibres lors de leur convoyage.

L'ouverture de la paroi du fourreau formée à l'alimentation 20 est avantageusement symétrique par rapport au rapport au plan vertical médian entre les axes 12A et 12B des vis pour assurer la bonne répartition des copeaux sur les deux vis dès leur entrée dans le fourreau. De même, l'ouverture formée à la sortie 22 du fourreau est avantageusement symétrique par rapport au même plan vertical.

Du fait de la rotation des vis, les copeaux sont entraînés dans le sens S allant de l'amont vers l'aval.

Dans la paroi inférieure du fourreau sont disposés un ou plusieurs filtres d'extraction 28 servant à l'extraction des jus provenant du défibrage ou les eaux de lavage des copeaux, permettant ainsi de réguler l'humidité finale du produit. Par exemple, ces filtres sont placés à l'extrémité amont des zones de freinage qui seront décrites dans la suite.

Les deux vis 12 et 14 tournent dans le même sens R et à la même vitesse de rotation. En effet, sur chaque tronçon des vis en vis-à-vis, les filets des deux vis sont de même sens.

Pour chaque vis, les filets présentent une série amont SM et une série aval SA de tronçons. En l'espèce, les filets présentent en outre une série intermédiaire SI située entre les série amont SM et aval SA. Ainsi, les séries SM, SI et SA sont disposées successivement depuis l'amont vers l'aval du fourreau.

Chacune de ces séries comprend elle-même une zone amont d'entraînement, respectivement SME, SIE et SAE pour les séries amont, intermédiaire et aval, ainsi qu'une zone aval de freinage, respectivement SMF, SIF et SAF pour les séries amont intermédiaire et aval. Ces zones d'entraînement et de freinage sont respectivement qualifiées de amont et de aval car, pour chaque série, la zone d'entraînement est en amont de la zone de freinage dans le sens S d'avancement des copeaux en cours de défibrage.

On voit que, dans les zones d'entraînement SME, SIE et SAE, les filets 12B et 14B des vis 12 et 14 sont à pas direct. Ceci signifie que, par la rotation des vis dans le sens R, ces filets font naturellement avancer vers l'aval le matériau qui est situé entre eux. En revanche, dans les zones de freinage SMF, SIF et SAF, les filets 12B et 14B sont à pas inverse, c'est-à-dire que la rotation des vis dans le sens R tend à faire reculer vers l'amont le matériau situé entre eux.

Il en résulte que, pour chaque série, le matériau en cours de défibrage a tendance à s'agglutiner à l'interface entre la zone d'entraînement et la zone de freinage. Pour permettre quand même l'acheminement du matériau vers l'aval à travers chaque zone de freinage, les filets des zones de freinage présentent des interruptions ou encoches 12C, 14C. Ainsi, ces encoches forment des zones d'étranglement à travers lesquelles le matériau est forcé à passer, sous l'effet de la poussée exercée, à l'amont, par le matériau entraîné vers l'aval par la zone d'entraînement amont.

Les encoches sont mieux visibles sur la figure 3 qui est une coupe verticale prise immédiatement à l'amont d'une zone de freinage (en l'espèce, la zone de freinage de la série amont SM) et montre l'organisation d'une zone de freinage. En l'espèce, pour la zone de freinage de chacune des deux vis 12 et 14, chaque filet comporte 5 encoches identiques, respectivement 12C et 14C réparties régulièrement angulairement.

Les axes géométriques des vis sont matérialisés par les références 12A et 14A, qui sont les axes de rotation de leurs arbres porteurs, respectivement 12P et 14P. Les tronçons de vis étant avantageusement démontables, leurs filets sont portés par des manchons, respectivement 12M et 14M, qui sont montés sur les arbres porteurs et leur sont solidarisés en rotation par tout moyen approprié, par exemple par des cannelures axiales (non représentées).

Pour chaque filet, les encoches sont délimitées radialement entre la périphérie radiale externe du filet et sa périphérie radiale interne, délimitée par la surface extérieure du manchon, respectivement 12M et 14M. Par exemple, le diamètre externe de chaque vis, délimité par la périphérie radiale externe de son filet, est de 240 mm, la hauteur radiale h d'une encoche est de 44mm et la largeur d'une encoche est de 16mm. Pour un filet, c'est-à-dire en suivant un filet de la vis sur un angle de 360°, on parvient ainsi à une somme des sections des encoches de ce filet de 5x44x16 = 3520 mm².

Avantageusement, dans une zone de freinage de la vis 12 ou 14, les encoches 12C ou 14C de deux filets consécutifs de la même vis sont légèrement décalées angulairement. Pour illustrer cette caractéristique sur la figure 3, on a représenté en traits épais les encoches des filets qui sont situés en premier à partir du plan de coupe, tandis qu'on a montré en trait fin la position des encoches qui équipent les filets situés immédiatement à l'aval de ces premiers filets. En l'espèce, le décalage angulaire est de l'ordre de 10 à 20 degrés et il est orienté dans le sens de rotation R des vis, de sorte qu'une ligne reliant deux encoches correspondantes de deux filets adjacents est orientée dans le même sens que les filets à pas direct.

L'alimentation de l'installation est réalisée en continu et le débit d'alimentation est réglé pour respecter les paramètres de pression et de température évoqués précédemment.

Ainsi, l'installation comprend avantageusement au moins un capteur de température CT situé à l'amont de la zone aval de freinage SMF de la série amont (dans la région du plan de coupe III-III). Une table de correspondance entre la température et la pression peut être établie. Ainsi, une élévation de la température révélée par le capteur de température CT peut révéler un risque d'élévation de pression trop important. Dans ce cas, l'installation peut être régulée en diminuant le débit d'alimentation en copeaux de bois. On peut également prévoir une mesure directe de la pression à l'aide d'un capteur de pression CP situé dans la même région que le capteur de température CT. Les mesures de ces capteurs (au moins celle du capteur de température CT) peuvent être fournies en entrée à un microprocesseur qui fournit une commande au système d'alimentation en copeaux de bois, par exemple une vis sans fin, comme indiqué précédemment. Si aucune mesure directe de pression n'est disponible, le microprocesseur peut, en mémoire, disposer d'une table de correspondance température/pression. Si une mesure directe de la pression est effectuée, le microprocesseur peut contrôler le système d'alimentation en copeaux de bois sur la base des deux données température/pression qui lui sont fournies. Pour une essence de bois déterminée et une humidité connue, il peut être établi une relation entre d'un part, les paramètres de pression et de température et, d'autre part, la puissance électrique consommée par le moteur qui entraîne les vis en rotation (ou l'intensité électrique délivrée, si la tension électrique est constante, comme c'est souvent le cas). Cette relation peut être déterminée de manière empirique par des essais. Cette relation étant connue, on peut obtenir les paramètres souhaités de pression et de température en réglant l'alimentation en copeaux de bois de manière à consommer une puissance cible.

Un aspect essentiel du procédé d'enrichissement par multiplication sur fibre de bois est la nécessité de refroidir la fibre de bois après sa fabrication. Le refroidissement doit s'effectuer dans des conditions qui préservent la propreté microbienne initiale.

Le bois qui passe dans les extrudeuses à vis ou dans les défibreuses à vapeur est chauffé jusqu'à des températures qui peuvent atteindre 150°C. A la sortie des machines, la détente et l'évaporation d'eau abaissent la température. Les mesures indiquent des températures de 50 à 80°C en sortie de machines. La température optimale d'incorporation des microorganismes dépend des espèces utilisées. Certaines bactéries thermophiles supportent sans difficulté des températures de 50 à 60°C, sans altération de la capacité de multiplication. Cela s'observe avec des bactéries telles les *Bacillus,* les *Paenibacillus.* Pour les autres microorganismes, des températures supérieures à 45°C sont critiques pour la survie des formes de résistance.

L'inoculation d'un substrat contenant des fibres de bois ne peut se faire qu'après avoir abaissé sa température en dessous de la température limite de croissance des microorganismes inoculés. Pour les *Trichoderma,* en général, la limite se situe à 37°C.

Le refroidissement de la fibre de bois demande, soit un passage dans un flux d'air filtré froid, soit une période de repos dans un contenant fermé pendant une période suffisante pour que la température soit inférieure à 37°C en tout point.

Le transport de la fibre de bois chaude sur une bande transporteuse placée dans un tunnel ventilé avec de l'air filtré est une méthode appropriée pour refroidir de grandes quantités de fibres chaudes stériles, sans risque de recontamination.

### Milieu de culture

Dans un mode réalisation de l'invention, le milieu de culture utilisé pour la préparation de l'inoculum primaire, et celui utilisé pour la préparation de l'inoculum secondaire, peuvent être stérilisés directement par passage dans une extrudeuse bivis, auquel cas aucune étape d'autoclavage n'est nécessaire. Dans ce mode de réalisation, des copeaux de bois, notamment des plaquettes de bois, sont extrudés dans une extrudeuse bivis, dans laquelle on introduit les différents constituants du milieu de culture (notamment les substances nutritives, le correcteur de pH et l'eau) sous forme de mélange liquide. La température d'extrusion est avantageusement de l'ordre de 120°C. On obtient ainsi un milieu de culture stérilisé.

### Multiplication de Trichoderma in situ

De manière générale, lors de la multiplication *in situ,* la production commence par une culture mère de microorganismes obtenue en laboratoire sur des milieux classiques de microbiologie. Cette culture mère sert à préparer un inoculum primaire, obtenu par croissance des microorganismes sur un milieu à base de fibres végétales « activées » par l'ajout de nutriments à base de glucides et de minéraux et convenablement désinfectées. Les microorganismes se multiplient entre 10⁴ et 10⁵ fois, en termes de nombre de propagules, au sein de l'inoculum primaire. L'inoculum primaire est un intermédiaire destiné soit à ensemencer des terreaux soit, de préférence, à produire un inoculum secondaire.

L'inoculum secondaire est à base de fibres végétales « activées » ou non en glucides et minéraux. L'inoculum secondaire doit être microbiologiquement propre, sans être nécessairement stérile. La multiplication dans l'inoculum secondaire est plus faible, 10³ à 10⁵ fois, en termes de nombre de propagules. Cet inoculum secondaire est lui-même destiné à ensemencer les terreaux.

Dans le cadre de l'invention, deux niveaux d'amplification des *Trichoderma* sont convenables pour atteindre un facteur de multiplication total de 200 000 à 1 000 000 entre la culture mère et le terreau fini.

La culture mère est préparée en boîtes de Pétri sur un milieu stérilisé, propre à la multiplication des champignons *Trichoderma.* Des milieux convenables sont le Potato Dextrose Broth (PDB), le Potato Dextrose Agar (PDA), le Malt Agar (MA ou MA2) ou le milieu de Mc Faden et Sutton au Rose Bengale / Streptomycine / Formol (RB-S-F).

L'inoculum primaire est préparé en laboratoire. La culture mère sert à préparer une suspension de propagules dans l'eau stérile. La suspension est incorporée dans le milieu de culture activé destiné à former l'inoculum primaire. Le milieu de culture activé est préparé avec des matières premières stérilisées. Il comprend des fibres de bois, et de préférence des substances nutritives, des correcteurs de pH et de l'eau. Si le milieu de culture n'est pas directement stérilisé comme indiqué ci-dessus, la stérilisation s'effectue par chauffage à haute température (121°C), sous pression de vapeur d'eau, dans un autoclave. L'incubation de l'inoculum primaire demande 2 à 3 semaines pour obtenir des propagules à l'optimum de leur viabilité. S'il n'est pas contaminé par d'autres microorganismes, l'inoculum primaire de *Trichoderma* se conserve à température inférieure à 22°C pendant plusieurs mois. La conservation à 4 à 6°C est encore meilleure. Dans l'inoculum primaire, la multiplication observée atteint couramment 2.10⁴ à 10⁵. La concentration finale en propagules se situe dans la gamme de 10⁸ à 10¹⁰ propagules/g.

L'inoculum secondaire est produit en atelier, dans un environnement propre mais non stérile, à partir de matières désinfectées par chauffage. Le défibrage des copeaux de bois dans un défibreur à vis (typiquement, une extrudeuse) qui produit un échauffement mécanique (100-120°C), ou dans un défibreur à disque sous pression de vapeur (3 à 8 bars) conduit à l'obtention de fibres de bois désinfectées. La fibre de bois doit être refroidie avant inoculation à une température compatible avec la survie du *Trichoderma,* donc moins de 37°C. Le milieu de culture (contenant les fibres de bois « désinfectées ») est lui-même désinfecté, par exemple par acidification et/ou ajout de sulfite (ou alternativement, par filtration stérilisante, rayonnement ultra-violet, chauffage). Il est également possible, comme pour la préparation de l'inoculum primaire, de désinfecter le milieu de culture par passage dans une extrudeuse bivis. L'incubation de l'inoculum secondaire demande 2 à 3 semaines à une température comprise entre environ 20°C et environ 25°C. Des essais de stabilité à une température de 22°C sur des périodes longue de 5 mois ont également été réalisés avec toujours une stabilité de la viabilité des spores de *Trichoderma.* La multiplication dans l'inoculum secondaire atteint en général 10³ à 10⁴. La concentration finale dans l'inoculum secondaire se situe dans la gamme de 10⁷ à 10⁹ propagules/g.

L'inoculum secondaire peut ensuite être incorporé dans des terreaux commerciaux à raison de 0,02 à 0,5 % m/m, soit environ 0,5 à 2 m³ d'inoculum secondaire / 1000 m³ terreau fini. L'inoculum secondaire peut être combiné avec des agents protecteurs de *Trichoderma* lors de son incorporation dans les terreaux. L'objectif est d'obtenir une concentration finale de 10⁴ à 10⁶ propagules/g dans le terreau fini. Le dosage optimal dépend de la souche de *Trichoderma* employée et des effets désirés. La concentration appropriée en *Trichoderma atroviride* MUCL45632 (décrite dans la demande de brevet EP-A-1 876 232) est 10⁵ propagules/g avec un minimum tolérable de 10⁴ propagules/g.

L'invention est illustrée par les exemples ci-après, donnés à titre purement indicatif.

### Exemple 1 : préparation d'un inoculum primaire

### Préparation de la suspension mère

On cultive le champignon *Trichoderma atroviride* MUCL45632 en boîte de Pétri sur un milieu approprié (PDA) pour qu'il produise des spores. On récupère les spores dans l'eau stérile en grattant la surface de la boite. On dose cette suspension en spores totales et on ajuste la concentration à 1.10⁷ spores/mL en spores totales de *Trichoderma* à l'aide d'eau stérile.

### Préparation du milieu de culture

a) On mélange 217 g de son de blé (utilisé pour l'alimentation animale) et 540 g de plaquettes de bois (matière sèche : 60-70 %) et on extrude ce mélange dans une extrudeuse bivis de type KRO 200 disponible auprès de la société Clextral, et dont les caractéristiques sont les suivantes :
   Longueur (mm) : 1600
   Zone compression : 2
   Rotation : Co
   Température : 120°C

Les fibres de bois présentent (après extrusion) une masse volumique apparente sèche de 40 kg/m³.
b) Dans un bêcher on mélange :

| | |
|---|---|
| eau | 722 g |
| vitamine C | 1,1 g |
| glycérol | 21,7 g |
| sulfite de sodium | 0,65 g |
| H₃PO₄ 84 % | 20,9 g |
| CoCl₂ | 0,003 g |

On verse la fraction liquide b) dans la fraction solide a) et on homogénéise. On verse le mélange dans un sac à autoclave en polypropylène de 2 L. Après le remplissage, le sac est scellé en laissant une ouverture de 7 cm bouchée par du coton cardé. Le sac est alors autoclavé pendant 35 min à 121°C.

*Après autoclavage :*
pH 1/5 vol : 3,1
Matière sèche : 32,8 %
Masse totale : 1523 g

### Ensemencement (inoculation) du milieu

150 mL de suspension mère de *Trichoderma* à 1.10⁷ spores/mL sont ajoutés au sac soit 1.10⁶ spores /g de milieu et on homogénéise.

### Culture

Le sac est placé dans une étuve régulée à 27°C pendant 4 jours, puis on maintient la température à 25°C. On plante dans le bas du sac à l'opposé de l'ouverture cotonnée, une aiguille reliée à une alimentation en air stérile sous pression par un tuyau pour apporter 10 L/h d'air pendant la culture. Après 7 jours, on mélange les sacs manuellement pour stimuler la sporulation. Au bout de 2 jours, les filaments du champignon commencent à apparaître. A 4 jours, le milieu de culture commence à s'échauffer. Une température de 28°C - 30°C est atteinte. On laisse la culture se dérouler pendant 21 jours.

### Observations en fin de culture

En fin de culture, on mesure :
pH 1/5 vol : 3,3
Matière sèche : 32 %

La viabilité et le degré de contamination du milieu de culture sont reportés dans le Tableau 1. Les spores viables sont dénombrées selon la norme NF ISO 7954.

**Tableau 1**

| Dosages | Spores totales/g | Propagules/g | Contaminants bactériens (UFC/g) |
|---|---|---|---|
| 21 jours de culture | 1,8.10⁹ | 1,0.10⁹ | 3,8.10⁴ |
| 1 mois stockage réfrigérateur | 2,0.10⁹ | 1,1.10⁹ | 3,3.10⁵ |
| 2 mois stockage réfrigérateur | 1,8.10⁹ | 1,2.10⁹ | nd |
| 3 mois stockage réfrigérateur | 1,5.10⁹ | 1,1.10⁹ | < 10⁴ |
| 1 mois stockage température ambiante | 2,1.10⁹ | 2,2.10⁹ | 2.10² |
| 2 mois stockage température ambiante | 2,3.10⁹ | 1,0.10⁹ | 6,8.10³ |
| 4 mois stockage température ambiante | 2,5.10⁹ | 1,4.10⁹ | nd |
| 6 mois stockage température ambiante | 1,3.10⁹ | 1,6.10⁹ | nd |

| | | | |
|---|---|---|---|
| nd = non déterminé | | | |

On constate à la lecture de ce Tableau que les lots conservent tous une viabilité supérieure ou égale à 10⁹ spores/g après 3 mois au réfrigérateur à 5 °C. On constate également que les lots conservent tous une viabilité supérieure ou égale à 10⁹ propagules/g après 6 mois à température ambiante. Il est préférable d'obtenir un produit sans contaminants, mais les bactéries présentes dans cet essai ne font pas chuter la viabilité du *Trichoderma.*

### Exemple 2 : préparation d'un inoculum secondaire

### Préparation du milieu de culture

a) On mélange 118 kg de son de blé (utilisé pour l'alimentation animale) et 481 kg de plaquettes de bois (matière sèche : 60-70 %) et on extrude ce mélange dans une extrudeuse bivis de type KRO 200 (cf. exemple 1).
b) Dans une cuve, on mélange:

| | |
|---|---|
| eau | 483 kg |
| vitamine C | 0,966 kg |
| sulfite de sodium | 0,240 kg |
| H₃PO₄ 84 % | 7,77 kg |

Le mélange doit être fait de façon à ne pas contaminer la future culture.

On verse la fraction liquide b) dans la fraction solide a) et on homogénéise dans un mélangeur.

### Ensemencement (inoculation) avec l'inoculum primaire

Dans 167 L d'eau propre, on rajoute 133 g de sulfite de sodium et le sac d'inoculum primaire de l'exemple précédent. On homogénéise la suspension et on laisse reposer pendant 24 h. On verse la suspension dans le mélangeur qui contient le milieu de culture et on mélange le tout. L'ensemencement se fait à 1.10⁶ spores totales/g de milieu.

### Culture

On verse le mélange dans 50 sacs en polypropylène de 25 kg scellés avec une ouverture cotonnée et une injection d'air. Ce système limite la perte d'eau. Alternativement, on peut utiliser 50 sacs en polypropylène tressés de 25 kg, mais le milieu se dessèche en cours de culture. Les sacs sont séparés les uns des autres pour éviter l'échauffement.

La culture est faite dans une salle propre close à une température comprise entre 19°C et 25°C. Les courants d'air sont évités et le passage du personnel est limité au strict nécessaire. La culture se déroule pendant 21 jours.

### Observations en fin de culture en sacs scellés

En fin de culture les fibres ont pris une couleur verte significative de la présence de spores de *Trichoderma.*

La viabilité et le degré de contamination du milieu de culture sont reportés dans le Tableau 2. Les spores viables sont dénombrées selon la norme NF ISO 7954.

**Tableau 2**

| Dosages | Spores totales/g | Propagules/g | Contaminants bactériens (UFC/g) |
|---|---|---|---|
| 21 jours de culture | 8,8.10⁸ | 1,1.10⁹ | 7,8.10⁴ |
| 1 mois stockage à 25 °C | 1,9.10⁹ | 1,2.10⁹ | 2,1.10⁵ |
| 2 mois stockage à 25 °C | 2,2.10⁹ | 1,1.10⁹ | nd |
| 5 mois stockage à 25 °C | 1,6.10⁹ | 1,0.10⁹ | nd |
| 7 mois stockage à 25 °C | 9,0.10⁸ | 6,7.10⁸ | nd |

| | | | |
|---|---|---|---|
| nd = non déterminé | | | |

### Observations en fin de culture en sacs tressés

En fin de culture, on mesure :
Matière sèche 38,9 %
pH : 3

La viabilité et le degré de contamination du milieu de culture sont reportés dans le Tableau 3. Les spores viables sont dénombrées selon la norme NF ISO 7954.

**Tableau 3**

| Dosage | Spores totales/g | Propagules/g | Contaminants bactériens (UFC/g) |
|---|---|---|---|
| 21 jours de culture | 9,7.10⁸ | 4.10⁸ | 9,5.10² |

### Exemple 3 : stabilité du Trichoderma dans les matières premières et le terreau

La stabilité de la viabilité des souche de *Trichoderma* est une propriété particulièrement remarquable qui répond aux attentes des utilisateurs qui recherchent des produits que l'on peut transporter et stocker sans risque de perte à température ambiante et sans le problème de respect de la chaîne du froid.

Les essais sur quelques matières premières et un terreau commercial montrent que les spores de *Trichoderma atroviride* MUCL45632 ne meurent pas lorsqu'ils sont cultivés, en présence des fibres de bois de l'invention, dans des conditions de conservation tempérées. Les résultats sont reportés dans le Tableau 4.

**Tableau 4**

| Dosages (propagules/g) | Théorique | Départ | 1 mois de stockage | 2 mois de stockage | 3 mois de stockage |
|---|---|---|---|---|---|
| fibre de l'invention | 5.10⁶ | 5.10⁶ | 1,5.10⁷ | 9,5.10⁶ | 4.10⁶ |
| tourbe blonde | 5.10⁶ | 6,3.10⁶ | 1,5.10⁶ | 1,1.10⁵ | 4,5.10⁴ |
| fibre de coco | 5.10⁶ | 1,7.10⁶ | 2,5.10⁷ | 1,7.10⁷ | 9,6.10⁶ |
| écorce fraîche | 5.10⁶ | 4,7.10⁶ | 2.10⁶ | 3.10⁵ | 6.10⁴ |
| écorce compostée | 5.10⁶ | 5,2_{.}10⁶ | 4,4.10⁵ | 3,5.10⁵ | 1,2.10⁵ |
| terreau commercial | 1.10⁷ | 5,8.10⁶ | 1.10⁶ | 1,1.10⁵ | 1,5.10⁴ |

## Revendications

1. Procédé de multiplication d'une souche de *Trichoderma,* qui comprend :
- l'ensemencement, par une suspension mère de propagules de *Trichoderma,* d'un premier milieu de culture stérilisé contenant entre environ 25% et environ 50% en masse de fibres de bois, de l'eau, des substances nutritives et un correcteur de pH, lesdites fibres ayant un taux d'air en volume compris dans la gamme allant d'environ 55% à environ 90%, ledit milieu étant exempt de contaminants fongiques et possédant un taux de contaminants bactériens inférieur ou égal à 10² UFC/g,
- la culture du milieu de culture ainsi ensemencé pour obtenir un inoculum primaire dans lequel le facteur de multiplication des propagules atteint 2.10⁴ à 10⁵.

2. Procédé de multiplication selon la revendication 1, dans lequel la concentration finale en propagules de l'inoculum primaire se situe dans la gamme de 10⁸ à 10¹⁰ propagules/g.

3. Procédé selon la revendication 1 ou la revendication 2, dans lequel la culture du milieu comprend une période d'incubation d'au moins deux semaines.

4. Procédé selon l'une quelconque des revendications 1 à 3, qui comprend en outre :
- la préparation d'un second milieu de culture contenant des fibres de bois désinfectées, lesdites fibres de bois ayant un taux d'air en volume compris dans la gamme allant d'environ 55% à environ 90% ;
- l'inoculation dudit second milieu de culture avec 0,001% à 5% en masse de l'inoculum primaire ;
- la culture du milieu de culture ainsi inoculé pour obtenir un inoculum secondaire dans lequel le facteur de multiplication des propagules atteint 10³ à 10⁵.

5. Procédé selon la revendication 4, dans lequel la concentration finale en propagules dans l'inoculum secondaire se situe dans la gamme de 10⁷ à 10⁹ propagules/g.

6. Procédé selon la revendication 4 ou la revendication 5, dans lequel la culture du milieu comprend une période d'incubation d'au moins deux semaines.

7. Procédé selon l'une quelconque des revendications 1 à 6, dans lequel les fibres de bois ont un taux d'air en volume compris dans la gamme allant d'environ 60% à environ 85%, de préférence d'environ 70% à environ 85%.

8. Procédé selon l'une quelconque des revendications 1 à 7, qui comprend une étape préalable d'extrusion de copeaux de bois, notamment de plaquettes de bois, à l'aide d'une extrudeuse bivis pour obtenir les fibres de bois destinées à être incorporées dans le premier milieu de culture et/ou le second milieu de culture.

9. Procédé selon la revendication 8, dans lequel les copeaux de bois sont extrudés en présence de son de blé.

10. Procédé selon l'une des revendications 8 et 9, dans lequel les fibres de bois sont susceptibles d'être obtenues par un procédé selon lequel on introduit des copeaux de bois (16) dans un fourreau de défibrage (10) contenant deux vis parallèles (12, 14) entraînées en rotation de manière à engrener l'une dans l'autre par leurs filets respectifs (12B, 14B), lesdits filets présentant successivement, dans le sens (S) allant de l'amont vers l'aval, au moins une série amont et une série aval de tronçons (SM, SI, SA) comprenant chacune une zone amont d'entraînement (SME, SIE, SAE) dans laquelle les copeaux sont entraînés vers l'aval et une zone aval de freinage (SMF, SIF, SAF) à travers laquelle les copeaux sont forcés sous l'effet d'entraînement procuré par la zone amont, les copeaux étant ainsi entraînés dans le fourreau (10) en étant freinés dans les zones de freinage et en étant transformés en fibres (24) que l'on récupère en sortie du fourreau de défibrage (10), la durée de séjour des copeaux dans le fourreau (10) étant comprise entre 15 et 80 secondes, une pression au moins sensiblement égale à 90 bars étant assurée à l'amont de la zone aval de freinage (SMF) de la série amont (SM), de telle sorte qu'une température comprise entre 120°C et 150°C soit atteinte dans le fourreau (10) sans apport extérieur de chaleur.

11. Procédé selon l'une quelconque des revendications 1 à 7, dans lequel le premier milieu de culture et/ou le second milieu de culture est (sont) stérilisé(s) par passage dans une extrudeuse bivis.

12. Procédé selon l'une quelconque des revendications 1 à 11, dans lequel la souche de *Trichoderma* est la souche *Trichoderma atroviride* MUCL45632.

13. Procédé de préparation d'un inoculum secondaire de *Trichoderma,* qui comprend :
- la préparation d'un milieu de culture contenant des fibres de bois désinfectées, lesdites fibres de bois ayant un taux d'air en volume compris dans la gamme allant d'environ 55% à environ 90% ;
- l'inoculation dudit milieu de culture avec 0,001% à 5% en masse d'un inoculum primaire de *Trichoderma* dont la concentration en propagules se situe dans la gamme allant de 10⁸ à 10¹⁰ propagules/g ;
- la culture du milieu de culture ainsi inoculé pour obtenir un inoculum secondaire dont la concentration en propagules se situe dans la gamme allant de 10⁷ à 10⁹ propagules/g.

14. Procédé selon la revendication 13, dans lequel la culture du milieu comprend une période d'incubation d'au moins deux semaines.

15. Procédé selon l'une des revendications 13 et 14, dans lequel les fibres de bois ont un taux d'air en volume compris dans la gamme d'environ 60% à environ 85%, de préférence d'environ 70% à environ 85%.

16. Procédé selon l'une quelconque des revendications 13 à 15, qui comprend une étape préalable d'extrusion de copeaux de bois, notamment de plaquettes de bois, à l'aide d'une extrudeuse bivis pour obtenir les fibres de bois destinées à être incorporées dans le milieu de culture.

17. Procédé selon la revendication 16, dans lequel les copeaux de bois sont extrudés en présence de son de blé.

18. Procédé selon l'une quelconque des revendications 13 à 15, dans lequel le milieu de culture est stérilisé par passage dans une extrudeuse bivis.

## Patentansprüche

1. Verfahren zur Vermehrung eines *Trichoderma*-Stammes, das umfasst:
- das Beimpfen eines ersten sterilisierten Kulturmediums, das zwischen etwa 25 und etwa 50 Masse-% Holzfasern, Wasser, Nährstoffe und einen pH-Korrektor enthält, mit einer Stammsuspension von *Trichoderma*-Propagulen, wobei die Fasern einen Luftvolumenanteil im Bereich von etwa 55 % bis etwa 90 % haben, wobei das Medium frei von Pilzkontaminanten ist und einen Anteil an Bakterienkontaminanten von weniger als oder gleich 10² UFC/g besitzt,
- das Kultivieren des so beimpften Kulturmediums, um einen Primärimpfstoff zu erhalten, bei dem der Vermehrungsfaktor der Propagulen 2.10⁴ bis 10⁵ erreicht.

2. Vermehrungsverfahren nach Anspruch 1, bei dem die Endkonzentration an Propagulen des Primärimpfstoffes im Bereich von 10⁸ bis 10¹⁰ Propagulen/g liegt.

3. Verfahren nach Anspruch 1 oder Anspruch 2, bei dem das Kultivieren des Mediums eine Inkubationszeit von wenigstens zwei Wochen umfasst.

4. Verfahren nach einem der Ansprüche 1 bis 3, das ferner umfasst:
- das Herstellen eines zweiten Kulturmediums, das desinfizierte Holzfasern umfasst, wobei die Holzfasern einen Luftvolumenanteil im Bereich von etwa 55 % bis etwa 90 % haben,
- das Beimpfen des zweiten Kulturmediums mit 0,001 bis 5 Masse-% des Primärimpfstoffes,
- das Kultivieren des so beimpften Kulturmediums, um einen Sekundärimpfstoff zu erhalten, bei dem der Vermehrungsfaktor der Propagulen 10³ bis 10⁵ erreicht.

5. Verfahren nach Anspruch 4, bei dem die Endkonzentration an Propagulen in dem Sekundärimpfstoff im Bereich von 10⁷ bis 10⁹ Propagulen/g liegt.

6. Verfahren nach Anspruch 4 oder Anspruch 5, bei dem das Kultivieren des Mediums eine Inkubationszeit von wenigstens zwei Wochen umfasst.

7. Verfahren nach einem der Ansprüche 1 bis 6, bei dem die Holzfasern einen Luftvolumenanteil im Bereich von etwa 60 % bis etwa 85 %, vorzugsweise von etwa 70 % bis etwa 85 % haben.

8. Verfahren nach einem der Ansprüche 1 bis 7, das einen vorherigen Schritt zum Extrudieren von Holzspänen, insbesondere von Hackschnitzeln, mit Hilfe eines Doppelschneckenextruders umfasst, um die Holzfasern zu erhalten, die dazu bestimmt sind, in das erste Kulturmedium und/oder das zweite Kulturmedium eingebettet zu werden.

9. Verfahren nach Anspruch 8, bei dem die Holzspäne in Gegenwart von Weizenkleie extrudiert werden.

10. Verfahren nach einem der Ansprüche 8 und 9, bei dem die Holzfasern geeignet sind, durch ein Verfahren erhalten zu werden, wonach Holzspäne (16) in eine Entfaserungshülse (10) eingebracht werden, die zwei parallele Schnecken (12, 14) enthält, welche drehangetrieben sind, so dass sie über ihre jeweiligen Gewinde (12B, 14B) ineinandergreifen, wobei die Gewinde in der Richtung (S) von stromauf nach stromab nacheinander wenigstens eine stromaufwärtige Reihe und eine stromabwärtige Reihe von Abschnitten (SM, SI, SA) aufweisen, die jeweils einen stromaufwärtigen Antriebsbereich (SME, SIE, SAE), in dem die Späne in stromabwärtiger Richtung mitgenommen werden, sowie einen stromabwärtigen Bremsbereich (SMF, SIF, SAF), durch den die Späne unter der durch den stromaufwärtigen Bereich verliehenen Antriebswirkung gepresst werden, umfassen, wodurch die Späne in der Hülse (10) unter Abbremsen in den Bremsbereichen und unter Umwandeln in Fasern (24), die am Ausgang der Entfaserungshülse (10) gewonnen werden, mitgenommen werden, wobei die Verweildauer der Späne in der Hülse (10) zwischen 15 und 80 Sekunden beträgt, wobei ein Druck von wenigstens im Wesentlichen gleich 90 bar stromauf des stromabwärtigen Bremsbereichs (SMF) der stromaufwärtigen Reihe (SM) derart sichergestellt wird, dass eine Temperatur zwischen 120 °C und 150 °C in der Hülse (10) ohne äußere Wärmezufuhr erreicht wird.

11. Verfahren nach einem der Ansprüche 1 bis 7, bei dem das erste Kulturmedium und/oder das zweite Kulturmedium durch Durchlaufen eines Doppelschneckenextruders sterilisiert wird (werden).

12. Verfahren nach einem der Ansprüche 1 bis 11, bei dem der *Trichoderma-*Stamm der *Trichoderma atroviride* Stamm MUCL45632 ist.

13. Verfahren zur Herstellung eines Sekundärimpfstoffes von *Trichoderma,* das umfasst:
- das Herstellen eines Kulturmediums, das desinfizierte Holzfasern umfasst, wobei die Holzfasern einen Luftvolumenanteil im Bereich von etwa 55 % bis etwa 90 % haben,
- das Beimpfen des Kulturmediums mit 0,001 bis 5 Masse-% eines Primärimpfstoffes von *Trichoderma,* dessen Konzentration an Propagulen im Bereich von 10⁸ bis 10¹⁰ Propagulen/g liegt,
- das Kultivieren des so beimpften Kulturmediums, um einen Sekundärimpfstoff zu erhalten, dessen Konzentration an Propagulen im Bereich von 10⁷ bis 10⁹ Propagulen/g liegt.

14. Verfahren nach Anspruch 13, bei dem das Kultivieren des Mediums eine Inkubationszeit von wenigstens zwei Wochen umfasst.

15. Verfahren nach einem der Ansprüche 13 und 14, bei dem die Holzfasern einen Luftvolumenanteil im Bereich von etwa 60 % bis etwa 85 %, vorzugsweise von etwa 70 % bis etwa 85 % haben.

16. Verfahren nach einem der Ansprüche 13 bis 15, das einen vorherigen Schritt zum Extrudieren von Holzspänen, insbesondere von Hackschnitzeln, mit Hilfe eines Doppelschneckenextruders umfasst, um die Holzfasern zu erhalten, die dazu bestimmt sind, in das Kulturmedium eingebettet zu werden.

17. Verfahren nach Anspruch 16, bei dem die Holzspäne in Gegenwart von Weizenkleie extrudiert werden.

18. Verfahren nach einem der Ansprüche 13 bis 15, bei dem das Kulturmedium durch Durchlaufen eines Doppelschneckenextruders sterilisiert wird.

## Claims

1. A method for multiplying a strain of *Trichoderma,* which comprises:
- the inoculating, with a stock suspension of *Trichoderma* propagules, of a first sterilized culture medium containing between about 25% and about 50% by weight of wood fibers, water, nutritive substances and a pH modifier, said fibers having an air content by volume in the range of from about 55% to about 90%, said medium being free of fungal contaminants and having a bacterial contaminant content of less than or equal to 10² CFU/g;
- the culturing of the culture medium thus inoculated so as to obtain a primary inoculum in which the multiplication factor of the propagules reaches 2 × 10⁴ to 10⁵.

2. The multiplication method as claimed in claim 1, in which the final concentration of propagules in the primary inoculum is in the range of from 10⁸ to 10¹⁰ propagules/g.

3. The method as claimed in claim 1 or claim 2, in which the culturing of the medium comprises an incubation period of at least two weeks.

4. The method as claimed in any one of claims 1 to 3, which further comprises:
- the preparation of a second culture medium containing wood fibers which have been disinfected, said wood fibers having an air content by volume included in the range of from about 55% to about 90%;
- the inoculation of said second culture medium with 0.001% to 5% by weight of the primary inoculum;
- the culturing of the culture medium thus inoculated so as to obtain a secondary inoculum in which the multiplication factor of the propagules reaches 10³ to 10⁵.

5. The method as claimed in claim 4, in which the final concentration of propagules in the secondary inoculum is in the range of from 10⁷ to 10⁹ propagules/g.

6. The method as claimed in claim 4 or claim 5, in which the culturing of the medium comprises an incubation period of at least two weeks.

7. The method as claimed in any one of claims 1 to 6, in which the wood fibers have an air content by volume in the range of from about 60% to about 85%, preferably from about 70% to about 85%.

8. The method as claimed in any one of claims 1 to 7, which comprises a prior step of extrusion of wood shavings, in particular of wood chips, using a twin-screw extruder so as to obtain the wood fibers intended to be incorporated into the first culture medium and/or the second culture medium.

9. The method as claimed in claim 8, in which the wood shavings are extruded in the presence of wheat bran.

10. The method as claimed in either of claims 8 and 9, in which the wood fibers can be obtained by a method according to which wood shavings (16) are introduced into a defiberizing barrel (10) containing two parallel screws (12, 14) driven rotationally so as to engage with one another via their respective threads (12B, 14B), said threads having successively, in the direction (S) from upstream to downstream, at least one upstream series and one downstream series of sections (SM, SI, SA) each comprising an upstream drive zone (SME, SIE, SAE) in which the shavings are driven in the downstream direction, and a downstream slowing down zone (SMF, SIF, SAF) through which the shavings are forced under the effect of drive provided by the upstream zone, these shavings thus being driven in the barrel (10) while being slowed down in the slowing down zones and while being converted into fibers (24) that can be recovered at the outlet of the defiberizing barrel (10), the residence time of the shavings in the barrel (10) being between 15 and 80 seconds, a pressure at least substantially equal to 90 bar being provided upstream of the downstream slowing down zone (SMF) of the upstream series (SM), such that a temperature of between 120°C and 150°C is reached in the barrel (10) without external provision of heat.

11. The method as claimed in any one of claims 1 to 7, in which the first culture medium and/or the second culture medium is (are) sterilized by being passed through a twin-screw extruder.

12. The method as claimed in any one of claims 1 to 11, in which the *Trichoderma* strain is the *Trichoderma atroviride* MUCL45632 strain.

13. A method for preparing a secondary inoculum of *Trichoderma,* which comprises:
- the preparation of a culture medium containing wood fibers which have been disinfected, said wood fibers having an air content by volume in the range of from about 55% to about 90%;
- the inoculation of said culture medium with 0.001% to 5% by weight of a primary inoculum of *Trichoderma,* the propagule concentration of which is in the range of from 10⁸ to 10¹⁰ propagules/g;
- the culturing of the culture medium thus inoculated so as to obtain a secondary inoculum, the propagule concentration of which is in the range of from 10⁷ to 10⁹ propagules/g.

14. The method as claimed in claim 13, in which the culturing of the medium comprises an incubation period of at least two weeks.

15. The method as claimed in either of claims 13 and 14, in which the wood fibers have an air content by volume in the range of from about 60% to about 85%, preferably about 70% to about 85%.

16. The method as claimed in any one of claims 13 to 15, which comprises a prior step of extrusion of wood shavings, in particular of wood chips, using a twin-screw extruder so as to obtain the wood fibers intended to be incorporated into the culture medium.

17. The method as claimed in claim 16, in which the wood shavings are extruded in the presence of wheat bran.

18. The method as claimed in any one of claims 13 to 15, in which the culture medium is sterilized by being passed through a twin-screw extruder.
